# EUROPEAN PATENT SPECIFICATION

(11) **EP 2 116 250 B1**
(45) Date of publication and mention of the grant of the patent: **20.06.2012**
(21) Application number: 09168004.1
(22) Date of filing: 21.12.2001
(51) Int. Cl.: A61K 31/565, A61P 5/24

(54) **Combination of an estrogen and an androgen for treating hormonal deficiencies in women undergoing estrogen replacement therapy**
Kombination von einem Östrogen und einem Androgen zur Behandlung von Hormonmangel in der Östrogenersatztherapie für Frauen
Combinaison d'un estrogène et d'un androgène pour le traitement de déficiences hormonales chez des femmes sous oestrogenothérapie substitutive

(30) Priority: 22.12.2000 US 258142 P
(43) Date of publication of application: 11.11.2009
(62) Divisional of application: 01989306.4
(73) Proprietor: Barr Laboratories, Inc., Woodcliff Lake, New Jersey 07677 (US)
(72) Inventor: Leonard, Thomas, W., Wilmington, NC 28405 (US); Waldon, R., Forrest, Wilmington, NC 28409 (US)
(74) Representative: Ward, Siobhan

(56) References cited:
- WO-A-00/42955
- WO-A-00/76522
- WO-A-94/26208

## Description

### Field of the Invention

The present invention generally relates to a pharmaceutical composition for use in a method of treating hormonal deficiencies in women, particularly during menopause.

### Background of the Invention

Menopause, which typically occurs in women during middle age, can be described as an ovarian shutdown. Commensurate therewith is a profound decrease in circulating levels of estrogens. There are a large variety of disorders and conditions attributed to the reduction of estrogen levels. Exemplary disorders and conditions include hot flashes, dryness and atrophy of the vagina, parathesia, dyspareunia, osteoporosis, and an increase in cardiovascular disease. Administration of estrogens to women, so-called "estrogen replacement therapy", to postmenopausal women continues to be the primary treatment of such disorders and conditions associated with menopause. Estrogens are also used in postmenopausal women in the treatment of osteoporosis and to delay onset of or prevent cardiovascular disease and Alzheimer's.

There is a distinct risk, in women with intact uteri, of developing endometrial hyperplasia from the administration of estrogen replacement therapy. The term "endometrial hyperplasia" refers to the overstimulation of the lining of the uterus which is a precursor to endometrial or uterine cancer. The development of endometrial hyperplasia is a significant issue with estrogen replacement therapy. It has been observed in U.S. Patent No. RE36,247. to Plunkett, et al., and U.S. Patent No. 5,043,331 to Hirvonen, that the co-administration of progestin can blunt the effect of estrogens. No one as yet has studies the effects of androgen or estrogen replacement on hyperplasia of the endometrium.

### Summary of the Invention

The present invention combines the administration of estrogens with the administration of non-aromatizing androgens, to have chronic estrogen therapy in postmenopausal women. We have generated data in an ovariectomized mouse model that demonstrates that estrogen/androgen therapy with an aromatizing androgen has a more detrimental effect on the uterus as evidenced by increased weight of the uterus than treatment with a non-aromatizing estrogen/androgen combination. One may also suspect that the use of an aromatizing androgen with an estrogen may also have negative effects in other tissues such as the breast. Therefore, an estrogen/androgen replacement therapy is best carried out with non-aromatizing androgens even in patients with and without intact uteri.

In one embodiment, the method of treating endometrial hyperplasia in a woman undergoing estrogen replacement therapy includes administering, continuously and uninterruptedly, a therapeutically effective amount of both estrogen and non-aromatizing androgen in daily dosages. In another embodiment, the method of treating includes cyclically administering the non-aromatizing estrogen compound and continuously and uninterruptedly administering the androgenic compound. A third embodiment utilizes continuously administering the estrogen compound with cyclic administering of the non-aromatizing androgenic compound.

### Brief Description of the Drawings

FIG. 1 is a graphical depiction of the weight of mice uterine horns on an estrogen, testosterone or a combination of estrogen and testosterone injection schedule.

FIG. 2 is a graphical depiction of the weight of mice uterine horns on an estrogen, oxandrolone or a combination of estrogen and oxandrolone injection schedule.

### Detailed Description of the Preferred Embodiments

The invention will now be described with reference to the embodiments set forth herein. These embodiments are intended to illustrate the invention and.are not meant to limit the scope of the invention.

In one aspect, the invention relates to a pharmaceutical composition for use in a method of treating endometrial hyperplasia in a woman undergoing estrogen replacement therapy. The pharmaceutical composition comprises a therapeutically effective amount of an estrogenic compound, a non-aromatizing androgenic compound, and a pharmaceutically acceptable carrier. A "therapeutically effective" amount as used herein is an amount of an estrogenic compound and a non-aromatizing androgenic compound that is sufficient to treat hormonal deficiencies in a subject. The therapeutically effective amount will vary with the age and physical condition of the patient, the severity of the treatment, the duration of the treatment, the nature of any concurrent treatment, the pharmaceutically acceptable carrier used and like factors within the knowledge and expertise of those skilled in the art. Pharmaceutically acceptable carriers are preferably solid dosage forms such as tablets or capsules, liquids, particularly aqueous, transdermal patches and other acceptable carriers, the selection of which are known in the art.

Estrogen levels are related to the general physiological health of postmenopausal women. They exert positive CNS effects on hot flashes, and improve nerve transmission which is believed to delay various types of dementia. They have positive cardiovascular effects by improving lipid levels and promoting vasodilation and relaxation. They also contribute to health of the vagina, provide local vasodilation effects and stimulate mucus production. Suitable estrogenic compounds include estrone, 17α-estradiol, 17β-estradiol, equilin, 17α-dihydroequilin, 17β-dihydroequilin, equilenin, 17α-dihydroequilenin, 17β-dihydroequilenin, Δ^{8,9} -dehydroestrone, 17α Δ^{8,9}-dehydroestradiol, 17β Δ^{8,9}-dehydroestradiol, 6-OH equilenin, 6-OH 17α-dihydroequilenin, ethinyl estradiol, estradiol valerate, 6-OH 17β-dihydroequilenin, and mixtures, conjugates and salts thereof, and the estrogen ketones and their corresponding 17α- and 17-β hydroxy derivatives. The estrogenic compounds may also be present as conjugated estrogens. The conjugates may be various conjugates understood by those skilled in the art, including, but not limited to, sulfate and glucuronide. The most preferred estrogen conjugates are estrogen sulfates. The estrogenic compounds may also be present as salts of estrogens conjugates. The salts may be various salts understood by those skilled in the art, including, but not limited to, sodium salts, calcium salts, magnesium salts, lithium salts, and piperazine salt. The most preferred salts are sodium salts. The estrogenic compounds can be derived from natural and synthetic sources. Preferably, the therapeutically effective amount of estrogenic compound is about 0.25 to about 3 mg, and preferably about 0.5 to about 2 mg based on oral dose equivalents of estradiol.

Suitable androgenic compounds include non-aromatizing compounds such as oxandrolone, oxymetholone, stanozolol, danazol, pharmaceutically acceptable esters and salts thereof, and combinations of any of the foregoing. Such androgenic compounds are commercially available from companies such as Steraloids Inc. and are found in their catalog "Steroids from Steraloids", 12 ed. Aromatizing androgenic compounds may produce estrogen and lead to estrogenic side effects. Non-aromatizing androgenic compounds often do not aromatize to estrogen. Preferably, the therapeutically effective amount of the non-aromatizing androgenic compound is about 0.25 to about 5 mg based on oral dose equivalents of oxandrolone.

The estrogen and androgen formulations can be, for example, in the form of tablets; effervescent tablets; pills; powders; elixirs; suspensions; emulsions; solutions; syrups; soft and hard gelatin capsules; transdermal patches; topical gels, creams and the like; vaginal suppositories; sterile injectable solutions; and sterile packaged powders, sublingual tablets, buccal tablets and buccal adhesive systems.

In certain embodiments, the drug product is present in a solid pharmaceutical composition that may be suitable for oral administration. A solid composition of matter according to the present invention may be formed and may be mixed with and/or diluted by an excipient. The solid composition of matter may also be enclosed within a carrier which may be, for example, in the form of a capsule, sachet, tablet, paper, or other container. When the excipient serves as a diluent, it may be a solid, semi-solid, or liquid material which acts as a vehicle, carrier, or medium for the composition of matter.

Various suitable excipients will be understood by those skilled in the art and may be found in the National Formulary, 19: 2404-2406 (2000), the disclosure of pages 2404 to 2406 being incorporated herein in their entirety. Examples of suitable excipients include, but are not limited to, starches, gum arabic, calcium silicate, microcrystalline cellulose, methacrylates, shellac, polyvinylpyrrolidone, cellulose, water, syrup, and methylcellulose. The drug product formulations can additionally include lubricating agents such as, for example, talc, magnesium stearate and mineral oil; wetting agents; emulsifying and suspending agents; preserving agents such as methyl- and propyl hydroxybenzoates; sweetening agents; or flavoring agents. Polyols, buffers, and inert fillers may also be used. Examples of polyols include, but are not limited to, mannitol, sorbitol, xylitol, sucrose, maltose, glucose, lactose, dextrose, and the like Suitable buffers encompass, but are not limited to, phosphate, citrate, tartarate, succinate, and the like. Other inert fillers which may be used encompass those which are known in the art and are useful in the manufacture of various dosage forms. If desired, the solid formulations may include other components such as bulking agents and/or granulating agents, and the like. The drug products of the invention may be formulated so as to provide quick, sustained, or delayed release of the active ingredient after administration to the patient by employing procedures well known in the art.

To form tablets for oral administration, the composition of matter of the present invention may be made by a direct compression process. In this process, the active drug ingredients may be mixed with a solid, pulverant carrier such as, for example, lactose, saccharose, sorbitol, mannitol, starch, amylopectin, cellulose derivatives or gelatin, and mixtures thereof, as well as with an antifriction agent such as, for example, magnesium stearate, calcium stearate, and polyethylene glycol waxes. The mixture may then be pressed into tablets using a machine with the appropriate punches and dies to obtain the desired tablet size. The operating parameters of the machine may be selected by the skilled artisan. Alternatively, tablets for oral administration may be formed by a wet granulation process. Active drug ingredients may be mixed with excipients and/or diluents. The solid substances may be ground or sieved to a desired particle size. A binding agent may be added to the drug. The binding agent may be suspended and homogenized in a suitable solvent. The active ingredient and auxiliary agents may also be mixed with the binding agent solution. The resulting dry mixture is moistened with the solution uniformly. The moistening typically causes the particles to aggregate slightly, and the resulting mass is pressed through a stainless steel sieve having a desired size. The mixture is then dried in controlled drying units for the determined length of time necessary to achieve a desired particle size and consistency The granules of the dried mixture are sieved to remove any powder. To this mixture, disintegrating, antifriction, and/or anti-adhesive agents are added. Finally, the mixture is pressed into tablets using a machine with the appropriate punches and dies to obtain the desired tablet size. The operating parameters of the machine may be selected by the skilled artisan.

If coated tablets are desired; the above prepared core may be coated with a concentrated solution of sugar or cellulosic polymers, which may contain gum arabic, gelatin, talc, titanium dioxide, or with a lacquer dissolved in a volatile organic solvent or a mixture of solvents. To this coating various dyes may be added in order to distinguish among tablets with different active compounds or with different amounts of the active compound present. In a particular embodiment, the active ingredient may be present in a core surrounded by one or more layers including enteric coating layers.

Soft gelatin capsules may be prepared in which capsules contain a mixture of the active ingredient and vegetable oil. Hard gelatin capsules may contain granules, of the active ingredient in combination with a solid, pulverulent carrier, such as, for example, lactose, saccharose, sorbitol, mannitol, potato starch, corn starch, amylopectin, cellulose derivatives, and/or gelatin.

In one preferred embodiment, the formulation is in the form of orally-administered tablets which contain the composition of matter of the present invention as set forth herein along with the following inactive ingredients: calcium phosphate tribasic, calcium sulfate, carnauba wax, cellulose, glyceryl monooleate, lactose, magnesium stearate, methylcellulose, pharmaceutical glaze, polyethylene glycol, stearic acid, sucrose, and titanium dioxide. Such ingredients may be present in amounts similar to those present in Premarin^{®} (conjugated, estrogens tablets, USP) made commercially available by. Wyeth-Ayerst Laboratories of Philadelphia, Pennsylvania. Tablets employing the active ingredients of the invention may contain excipients similar to those contained in the 0.3 mg, 0.625 mg, and 1.25 mg tablets of Premarin^{®} (conjugated estrogens tablets, USP).

Liquid preparations for oral administration may be prepared in the form of syrups or suspensions, e.g., solutions containing an active ingredient, sugar, and a mixture of ethanol, water, glycerol, and propylene glycol. If desired, such liquid preparations may contain coloring agents, flavoring agents, and saccharin. Thickening agents such as carboxymethylcellulose may also be used.

In the event that the above formulations are to be used for parenteral administration, such a formulation may comprise sterile aqueous injection solutions, non-aqueous injection solutions, or both comprising the composition of matter of the present invention, When aqueous injection solutions are prepared, the composition of matter may be present as a water soluble pharmaceutically acceptable salt. Parenteral preparations may contain anti-oxidants, buffers, bacteriostats, and solutes which render the formulation isotonic with the blood of the intended recipient. Aqueous and non-aqueous sterile suspensions may include suspending agents and thickening agents. The formulations may be presented in unit-dose or multi-dose containers, for example sealed ampules and vials. Extemporaneous injection solutions and suspensions may be prepared from sterile powders, granules and tablets of the kind previously described.

In a preferred embodiment, the drug product of the present invention is in the form of an injectable solution containing a predetermined amount (e.g., 25 mg) of the composition of matter in a sterile lyophilized cake which also contains lactose, sodium' citrate, and simethicone. The pH of a solution containing the above ingredients may be adjusted using a suitable buffer (e.g., sodium hydroxide or hydrochloric acid). Reconstitution may be carried out according to known methods, e.g., using a sterile diluent (5 mL) containing 2 percent by volume benzyl alcohol in sterile water. A preferred injectable solution is similar to Premarin^{®} Intravenous made commercially available by Wyeth-Ayerst Laboratories.

The composition of matter also may be formulated such that it is suitable for topical administration (e.g., vaginal cream). These formulations may contain various excipients known to those skilled in the art. Suitable excipients may include, but are not limited to, cetyl esters wax, cetyl alcohol, white wax, glyceryl monostearate, propylene glycol, monostearate, methyl stearate, benzyl alcohol, sodium lauryl sulfate, glycerin, mineral oil, water, carbomer, ethyl alcohol, acrylate adhesives, polyisobutylene adhesives, and silicone adhesives.

In a preferred embodiment, the drug product is in the form of a vaginal cream containing the composition of matter as set forth herein present in a nonliquefying base. The nonliquefying base may contain various inactive ingredients such as, for example, cetyl esters wax, cetyl alcohol, white wax, glyceryl monostearate, propylene glycol, monostearate, methyl stearate, benzyl alcohol, sodium lauryl sulfate, glycerin; and mineral oil. Such composition may be formulated similar to Premarin^{®} Vaginal Cream made commercially available by Wyeth-Ayerst Laboratories.

Dosage units for vaginal or rectal administration may be prepared in the form of suppositories which may contain the composition of matter in a mixture with a neutral fat base polyethylene glycol, or they may be prepared in the form of gelatin-rectal capsules which contain the active substance in a mixture with a vegetable oil or paraffin oil.

The present invention is explained in greater detail in the Examples which follow, These examples are intended as illustrative of the invention and are not to be taken are limiting thereof.

### EXAMPLES

### MATERIALS AND METHODS

Applicants performed a set of experiments to determine the effects of estrogen and testosterone on weight of uterine horns in mice.

The protocol employed in the experiment included taking ovariectomized female mice ordered from Charles River Laboratories, of which the females were 32-52 days old, after ovariectomy, and separating them into four groups:, The first group of mice received daily injections of testosterone for 7 days. A second group of mice received daily injections of estradiol for 7 days. A third group of mice received daily injections of testosterone plus estradiol for 7 days. Finally, the mice in the fourth group received daily injections of a control vehicle for 7 days. All of the injections were given IM at 10AM, alternatively to the right and left buttock. The daily amount injected of the estradiol and/or testosterone was 10µg/Kg of each hormone, delivered at a dose of 0.25 ml.

The injections were produced based upon the following protocols. The estradiol selected was the oil-dissolved 17β-estradiol from Sigma (17β-estradiol, E-8875. The estradiol was prepared by dissolving 200 µg of the hormone in 0.25 ml ethanol for a x1000 stock (0.8mg/1ml = x1000 stock). Next, 10µl aliquots were dispensed into nine 15ml conical plastic tubes and frozen. For each injection, one of the tubes was thawed, 9.99ml saline was added and then mixed to obtain 10ml of the solution at a level of 0.8µg/ml. Then, 0.25ml were injected into each animal.

The testosterone selected was testosterone enanthane from Bristol Myers Squib (Delatestryl ^{™}). The testosterone was prepared by aspirating 0.15 ml from a bottle containing 200mg/1ml testosterone via a tuberculin syringe of 1.0 ml. Next, 24.9ml of ethanol was poured into a 50ml conical plastic tube. Then 0.1ml of the testosterone was dissolved in the syringe with the 24.9ml of ethanol. This produced a x1000 stock of 0.8mg/1ml. Similar to the estradiol protocol, next, 10µl aliquots were dispensed into nine 15ml conical plastic tubes and frozen. For each injection, one of the tubes was thawed, 9.99ml saline is added and then mixed to obtain 10ml of the solution at a level of 0.8µg/1ml. Then, 0.25ml were injected into each animal. The injections were based for a 20 gm animal. The dose injected per actual weight of the animal may be adjusted by varying the weight, i.e., may vary from 19gm to 24gm (0.238ml to 0.3ml).

After the seven days of injections, the mice were euthanized via injection from Lumina. The mice were then weighed. After midline laparotomy, 2ml to 5ml does of blood was obtained from the heart. The blood was allowed to clot and was then centrifuged and the resulting serum was collected and stored at -80°C. The uterine horns were then identified, transected from the level of the vagina till the ovary (without the ovary and without the parametrium), weighted (*i.e*., wet weight of Rt and Lt horn), and placed separately in a piece of silver foil paper, and baked in an oven at 90°C for 24 hrs to obtain the dry weight of the horns.

Table 1 illustrates the data obtained from this experiment.

**[0041] Table 1**

| Group | BW (gm) | Rt H | Lt H | W Hs/BW | RtH | Lt H | D Hs / BW |
|---|---|---|---|---|---|---|---|
| | | W(mg) | W(mg) | (mg/gm) | D(mg) | D(mg) | (mg/gm) |
| | | | | | | | |
| | | | | | | | |
| C | 23.13 | 8.5 | 4.1 | 0.55 | 1.3 | 1.6 | 0.13 |
| C | 21.53 | 2.4 | 5.3 | 0.36 | 0.7 | 1.6 | 0.11 |
| C | 20.08 | 9.8 | 13.9 | 1.18 | 2.5 | 3.6 | 0.30 |
| C | 23.58 | 10.3 | 11.4 | 0.92 | 2.7 | 3.0 | 0.24 |
| C | 22.72 | 27.7 | 14.1 | 1.84 | 4.4 | 3.4 | 0.34 |
| Mean | 22.2 | 11.7 | 9.8 | 0.97 | 2.3 | 2.6 | 0.22 |
| SD | 1.4 | 9.5 | 4.8 | 0.58 | 1.4 | 1.0 | 0.10 |
| | | | | | | | |
| | | | | | | | |
| T | 21.77 | 16.2 | 21.3 | 1.72 | 4.3 | 8.2 | 0.57 |
| T | 21.68 | 11.7 | 15.6 | 1.26 | 1.1 | 4.3 | 0.25 |
| T | 21.22 | 13.5 | 3.7 | 0.81 | 2.5 | 0.8 | 0.16 |
| T | 23.25 | 8.4 | 8.4 | 0.72 | 1.1 | 0.9 | 0.09 |
| T | 21.89 | 20.1 | 13.9 | 1.55 | 4.8 | 1.9 | 0.31 |
| Mean | 22.0 | 14.0 | 12.6 | 1.21 | 2.8 | 3.2 | 0.27 |
| SD | 0.8 | 4.4 | 6.8 | 0.44 | 1.7 | 3.1 | 0.19 |
| | | | | | | | |
| | | | | | | | |
| E | 21.89 | 20.6 | 21.5 | 1.92 | 3.3 | 2.9 | 0.28 |
| E | 22.06 | 16.2 | 18.0 | 1.55 | 2.5 | 2.2 | 0.21 |
| E | 21.47 | 26.6 | 24.2 | 2.37 | 4.0 | 4.2 | 0.38 |
| E | 20.55 | 17.3 | 19.5 | 1.79 | 2.4 | 2.6 | 0.24 |
| E | 22.86 | 16.9 | 16.7 | 1.47 | 2.6 | 2.4 | 0.22 |
| Mean | 21.8 | 19.5 | 20.0 | 1.82 | 3.0 | 2.9 | 0.27 |
| SD | 0.8 | 4.3 | 2.9 | 0.36 | 0.7 | 0.8 | 0.07 |
| | | | | | | | |
| | | | | | | | |
| E+T | 21.15 | 53.2 | 53.0 | 5.02 | 8.2 | 6.7 | 0.70 |
| E+T | 21.3 | 25.6 | 24.0 | 2.33 | 3 | 4.6 | 0.36 |
| E+T | 20.52 | 40.5 | 21.0 | 3.00 | 5.5 | 3.5 | 0.44 |
| E+T | 22.06 | 24.2 | 29.3 | 2.43 | 4.8 | 5 | 0.44 |
| E+T | 20.8 | 27.6 | 17.2 | 2.15 | 5.8 | 2.9 | 0.42 |
| Mean | 21.2 | 34.2 | 28.9 | 2.99 | 5.5 | 4.5 | 0.47 |
| SD | 0.6 | 12.4 | 14.2 | 1.18 | 1.9 | 1.5 | 0.13 |

| | | | | | | | |
|---|---|---|---|---|---|---|---|
| Abbreviations are as follows: C - control, T - testosterone, E - estrogen, BW - body weight, Rt - right, Lt-left, H-horn, W - wet, D - dry, Hs - horns (Rt+Lt) | | | | | | | |

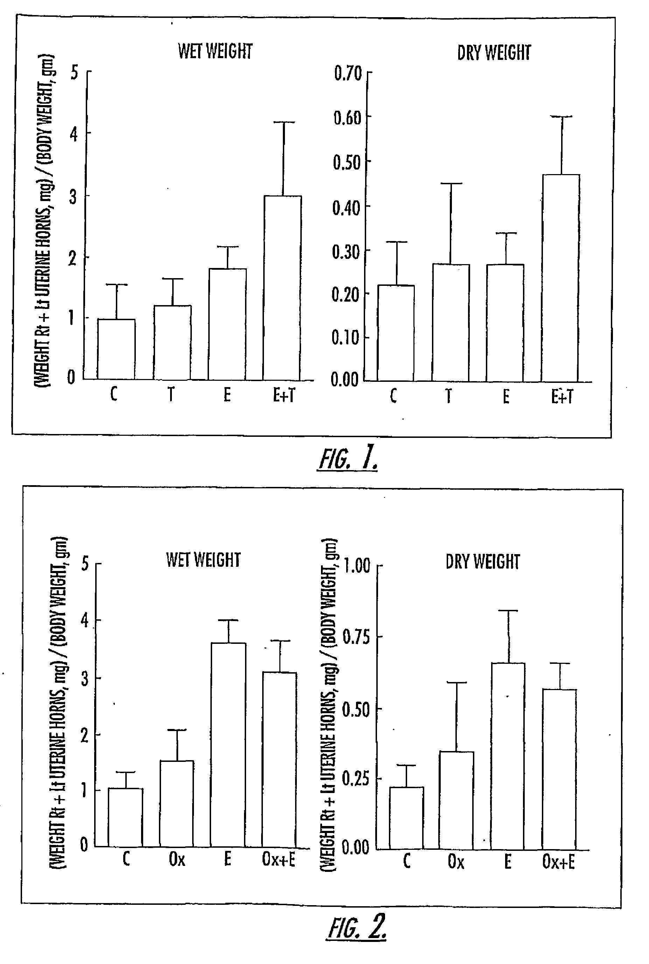

A graphical depiction of table 1 is found in FIG. 1. This figure illustrates that the testosterone accompanied by the estradiol produces an additive effect. This additive effect is a negative effect for endometrial hyperplasia. This means that the negative effects of estrogen on the uterus, and perhaps, on other tissues such as breast tissue, are magnified by the co-administering of an aromatic androgen, such as testosterone. It is not known whether progestin will obviate this effect as it does when only estrogen has been administered.

A second experiment was performed using the protocols set above. The only change included replacing testosterone with oxandrolone. The oxandrolone was prepared by dissolving a 20 mg powder of the original vial in 1.0 ml of DMSO. 100µl aliquots were then dispensed into 10 new vials (each containing now 2mg/vial, or 20µg/µl) and all vials, except one, were frozen at -20°C. The 9 frozen vials are referred to herein as "Original Stocks".

Next, one of the Original Stock vials containing 100µl of 20µg/µl Oxandrolone was diluted to a 1:5 ratio in DMSO by adding 400µl DMSO. This results in of a 4µg/µl Oxandrolone solution. 50µl aliquots were then dispensed into 10 vials (each containing now 0.4µg/µl, or 20µg/50µl) of which nine were frozen. The frozen vials from this part of the experiment are herein referred to as "Diluted Stocks".

One Diluted Stock vial was taken and diluted in 24.95ml saline to get a solution of 20µg/25ml oxandrolone, or 0.2µg/0.25ml. The 0.5ml aliquots are then dispensed into 20 vials and frozen. The remaining 15ml were discarded. On the day of injection, each one of the vials was thawed 0.25ml were injected into each animal based upon a 20gm animal. The dose injected may be adjusted per actual weight of the animal (may vary from 19gm to 24gm, 0.238ml to 0.3ml).

After the last day of injections, the mice were weighed and then euthanized in a CO₂ chamber. Blood was obtained from the heart in an amount of 2-5 ml. Time was allotted to let it clot and then it was centrifuged and the serum was collected and stored at - 80°C. The abdomen was opened by a longitudinal ventral incision. Then the uterine horns were identified and the right horn was transected from the level of the vagina until the ovary (thus, without the ovary and without the parametrium). This process was repeated for the left horn. Both horns were weighed to determine the wet weight of the horns. Then the horns were placed on silver foil paper and baked in an oven at 90°C for 24 hrs. Then the dry weight of the horns was taken.

Table 2 illustrates the results of this experiment.

**[0048] Table 2**

| Group | B W (gm) | Rt H | Lt H | W Hs/BW | Rt H | Lt H | XD Hs/BW |
|---|---|---|---|---|---|---|---|
| | | W(mg) | W(mg) | (mg/gm) | D(mg) | D(mg) | (mg/gm) |
| | | | | | | | |
| C | 22.18 | 11.0 | 10.8 | 0.98 | 1.1 | 2.7 | 0.17 |
| C | 21.18 | 11.7 | 5.4 | 0.81 | 1.4 | 2.1 | 0.17 |
| C | 21.52 | 8.6 | 14.0 | 1.05 | 1.8 | 3.1 | 0.23 |
| C | 22.11 | 9.1 | 9.7 | 0.85 | 1.6 | 2.4 | 0.18 |
| C | 21.84 | 18.4 | 15.3 | 1.54 | 4.7 | 3.1 | 0.36 |
| Mean | | | | 1.05 | | | 0.22 |
| SD | | | | 0.29 | | | 0.08 |
| | | | | | | | |
| B | 22.56 | 46.7 | 44.9 | 4.06 | 9.9 | 12 | 0.97 |
| E | 20.17 | 41.2 | 29.7 | 3.52 | 7.3 | 4.8 | 0.60 |
| E | 20.42 | 30 | 48.1 | 3.82 | 4.7 | 7.7 | 0.61 |
| E | 22.72 | 35 | 34.4 | 3.05 | 6.5 | 4.6 | 0.49 |
| E | 20.78 | 44.2 | 32.7 | 3.70 | 8.0 | 5 | 0.63 |
| Mean | | | | 3.63 | | | 0.66 |
| SD | | | | 0.38 | | | 0.18 |
| | | | | | | | |
| Ox | 22.04 | 16.5 | 14.4 | 1.40 | 3.4 | 2.3 | 0.26 |
| Ox | 21.13 | 12.0 | 13.8 | 1.22 | 2.0 | 2.2 | 0.20 |
| Ox | 23.76 | 35.7 | 22.9 | 2.47 | 12.5 | 5.5 | 0.76 |
| Ox | 22.08 | 13.2 | 11.3 | 1.11 | 2.2 | 1.4 | 0.16 |
| Ox | 22.69 | 14.6 | 19.4 | 1.50 | 4.2 | 4.3 | 0.37 |
| Mean | | | | 1.54 | | | 0.35 |
| SD | | | | 0.54 | | | 0.24 |
| | | | | | | | |
| E+Ox | 21.68 | | 20.3 | 2.53 | 7.5 | 2.9 | 0.48 |
| E+Ox | 22.89 | 41.3 | 27.9 | 3.02 | 10.1 | 6.2 | 0.71 |
| E+Ox' | 21.71 | 32 | 54.2 | 3.97 | 6.5 | 5.8 | 0.57 |
| E+Ox | 20.33 | 35.1 | 31.1 | 3.26 | 5.5 | 6.2 | 0.58 |
| E+Ox | 20.82 | 32.7 | 25.2 | 2.78 | 7.1 | 3.9 | 0.53 |
| Mean | | | 3.11 | | | | 0.57 |
| SD | | | 0.55 | | | | 0.09 |

| | | | | | | | |
|---|---|---|---|---|---|---|---|
| Abbreviations are as follows: C - control, Ox - Oxandrolone, E - estrogen, BW - body weight, Rt - right, Lt - left, H-horn, W - wet, D - dry, Hs - horns (Rt+Lt) | | | | | | | |

A graphical depiction of table 2 is found in FIG. 2, FIG. 2 demonstrates that an estradiol plus oxandrolone does not provide an additive effect. Instead the result is a reduction effect. Thus, the combination of an estradiol with oxandrolone is useful in treating hormonal deficiencies in women in need thereof.

. These examples are suitable for mice and other animals who have difficultly in swallowing tablets. For use in humans, the preferred embodiments will be tablets, capsules, transdermal pitches and the like.

## Claims

1. A pharmaceutical composition for use in the treatment of endometrial hyperplasia in a woman undergoing estrogen replacement therapy, said composition comprising:
a therapeutically effective amount of an estrogenic compound;
a therapeutically effective amount of a non-aromatizing androgenic compound; and
a pharmaceutically acceptable carrier.

2. A pharmaceutical composition according to claim 1 wherein the estrogenic compound is selected from the group consisting of estrone, 17α-estradiol, 17β-estradiol, equilin, estriol, 17α-dihydroequilin, 17β-dihydroequilin, equilenin, 17α-dihydroequilenin, 17β-dihydroequilenin, Δ^{8,9} -dehydroestrone, 17α-Δ^{8,9}-dehydroestradiol, 17β-Δ^{8,9}-dehydroestradiol, ethinyl estradiol, estradiol valerate, 6-OH equilenin, 6-OH 17α-dihydroequilenin, 6-OH 17-dihydroequilenin, and mixtures, conjugates and salts thereof.

3. A pharmaceutical composition according to claim 1 or claim 2 wherein the non-aromatizing androgenic compound is selected from the group consisting of oxandrolone, oxymetholone, stanozolol, danazol, pharmaceutically acceptable esters and salts thereof, and combinations of any of the foregoing.

4. The pharmaceutical composition of any of claims 1 to 3 further comprising a therapeutically effective amount of a progestin compound.

## Patentansprüche

1. Pharmazeutische Zusammensetzung zum Einsatz in der Behandlung von endometrialer Hyperplasie bei einer Frau, die sich in Östrogenersatztherapie befindet, wobei die genannte Zusammensetzung folgendes umfasst:
eine therapeutisch wirksame Menge einer Östrogenverbindung;
eine therapeutisch wirksame Menge einer nicht aromatisierenden androgenen Verbindung; und
einen pharmazeutisch zulässigen Träger.

2. Pharmazeutische Zusammensetzung nach Anspruch 1, wobei die Östrogenverbindung aus der Gruppe ausgewählt wird, die folgendes umfasst: Östron, du 17α-Östradiol, 17β-Östradiol, Equilin, Östriol, 17α-Dihydroequilin, 17β-Dihydroequilin, Equilenin, 17α-Dihydroequilenin, 17β-Dihydroequilenin, Δ^{8,9}-Dehydroöstron, 17α-Δ^{8,9}-Dehydroöstradiol, 17β-Δ^{8,9}-Dehydroöstradiol, Ethinylöstradiol, Östradiolvalerat, 6-OH-Equilenin, 6-OH 17α-Dihydroequilenin, 6-OH 17β-Dihydroequilenin sowie Mischungen, Konjugate und Salze dieser.

3. Pharmazeutische Zusammensetzung nach Anspruch 1 oder Anspruch 2, wobei die nicht aromatisierende androgene Verbindung aus der Gruppe ausgewählt wird, die folgendes umfasst: Oxandrolon, Oxymetholon, Stanozolol, Danazol, pharmazeutisch zulässige Ester und Salze dieser sowie alle Kombinationen der vorstehenden Stoffe.

4. Pharmazeutische Zusammensetzung nach einem der Ansprüche 1 bis 3, wobei diese ferner eine therapeutisch wirksame Menge einer Progestinverbindung umfasst.

## Revendications

1. Composition pharmaceutique destinée à être utilisée dans le traitement de l'hyperplasie endométriale chez une femme subissant une oestrogénothérapie de substitution, ladite composition comprenant :
une quantité efficace du point de vue thérapeutique d'un composé oestrogénique ;
une quantité efficace du point de vue thérapeutique d'un composé androgénique non aromatisant ; et
un support pharmaceutiquement acceptable.

2. Composition pharmaceutique selon la revendication 1, dans laquelle le composé oestrogénique est sélectionné parmi le groupe constitué de l'estrone, du 17α-estradiol, du 17β-estradiol, de l'équiline, de l'estriol, de la 17α-dihydroéquiline, de la 17β-dihydroéquiline, de l'équilénine, de la 17β-dihydroéquilénine, de la 17β-dihydroéquilénine, de la Δ^{8,9}-déshydroestrone, du 17α-Δ^{8,9}-déshydroestradiol, du 17β-Δ^{8,9}-déshydroestradiol, de l'éthinylestradiol, du valérate d'estradiol, de la 6-OH équilénine, de la 6-OH 17α-dihydroéquilénine, de la 6-OH 17β-dihydroéquilénine et de mélanges, conjugués et sels de ceux-ci.

3. Composition pharmaceutique selon la revendication 1 ou la revendication 2, dans laquelle le composé androgénique non aromatisant est sélectionné parmi le groupe constitué de l'oxandrolone, de l'oxymétholone, du stanozolol, du danazol, d'esters et de sels pharmaceutiquement acceptables de ceux-ci et de combinaisons de l'un quelconque des composés précédents.

4. Composition pharmaceutique selon l'une quelconque des revendications 1 à 3, comprenant en outre une quantité efficace du point de vue thérapeutique d'un composé de progestine.
